# EUROPEAN PATENT APPLICATION

(11) **EP 3 967 369 A1**
(43) Date of publication of application: **16.03.2022**
(21) Application number: 19928554.5
(22) Date of filing: 10.05.2019
(51) Int. Cl.: A61P 19/08, A61P 1/02

(54) **DENTAL ROOT CANAL FILLING MATERIAL COMPOSITION**

(71) Applicant: Nippon Shika Yakuhin Co., Ltd., Yamaguchi 750-0015 (JP)
(72) Inventor: YOKOTA Kazuyoshi, Shimonoseki-shi Yamaguchi 750-0015 (JP); SAKANO Emi, Shimonoseki-shi Yamaguchi 750-0015 (JP); ITOH Shousaku, Suita-shi, Osaka 565-0871 (JP); HAYASHI Mikako, Suita-shi, Osaka 565-0871 (JP)
(74) Representative: Lederer & Keller Patentanwälte Partnerschaft mbB
(86) International application number: PCT/JP2019/018754
(87) International publication number: WO 2020/230192

(57) **Abstract**

A root canal filling material composition comprising a lithium salt has higher healing promoting activity than conventional root canal filling material compositions. Preferable compositions are (1) a composition further comprising a calcium hydroxide, (2) a combination composition of a paste A containing a higher fatty acid and rosin and a paste B containing magnesium oxide and a vegetable oil, and one or both of the paste A and the paste B comprise the lithium salt, and (3) a combination composition of a paste A and a paste B and hardens by mixing the paste A and the paste B, one or both of the paste A and the paste B comprise the lithium salt, one or both of the paste A and the paste B comprise a glass powder, the glass powder contains calcium oxide and silicon dioxide in a total amount of 50 to 100% by weight relative to a total amount of the glass powder, and in the glass powder, a weight ratio of the calcium oxide to the silicon dioxide (calcium oxide: silicon dioxide) is 6:4 to 3:7.

## Description

### Technical Field

The present invention relates to a dental root canal filling material composition.

### Background Art

The root canal of a tooth has a dental pulp therein including nerves and blood vessels. When a dental pulp is infected or inflamed due to a deep dental caries, a fractured tooth crown, or the like and is allowed to go untreated, an abscess may be formed at the root apex of the root canal, the inflammation may spread in the periapical tissue, the gum may swell, or general symptoms such as swollen lymph nodes and fever may develop in some cases. Hence, when a dental pulp is infected or inflamed, the dental pulp is removed as needed, then the inside of the root canal is disinfected, and the dental pulp cavity is sealed with a root canal filling material to block stimuli to the periapical tissue.

Conventional root canal disinfectants contain active components such as formalin, paraformaldehyde, parachlorophenol, guaiacol, eugenol, zinc oxide, and calcium hydroxide.

Formalin and paraformaldehyde cause coagulation necrosis of protein, thus function to protect the internal tissue, and also have very strong bactericidal activity or disinfectant activity. Parachlorophenol, guaiacol, and eugenol have disinfectant activity and strong analgesic activity. Zinc oxide has disinfectant activity and anti-inflammatory activity. These components, however, have no activity to promote the healing of a root canal-periapical tissue.

In contrast, calcium hydroxide has disinfectant activity and analgesic activity and is believed to have an activity to promote healing of the root canal-periapical tissue. Hence, in Europe and America, calcium hydroxide has been commonly used as an active component in infected root canal therapeutic agents, and in Japan, dental treatment preparations containing calcium hydroxide have been developed and improved.

The application development of calcium hydroxide in the dental treatment preparations will be described in detail. Calcium hydroxide is a white powder, and an aqueous solution of calcium hydroxide has a pH of 12.6, which is strong alkaline, and thus has a strong disinfectant activity. Calcium hydroxide also has an activity to promote the formation of hard tissue of dental enamel, dentine, and dental cementum and thus has been contained in pulp capping materials, vital pulpotomy materials, root canal filling materials, and similar materials (Dental Outlook, 72(1), 63-74, 1988; The Journal of Japan Endodontic Association, 16(1), 47-51, 1995; and The Japanese Journal of Pediatric Dentistry, 26(3), 611-620, 1988).

Calcium hydroxide has also been widely used for apexification of a tooth with an immature root canal, sealing of a perforated site, and similar purposes (The Journal of Japan Endodontic Association, 14(2), 89-95, 1993; The Journal of Japan Endodontic Association, 16(1), 70-73, 1995; and The Japanese Journal of Pediatric Dentistry, 24(3), 459-467, 1986) .

Calcium hydroxide is also used actively as a short-term disinfectant (temporary root canal filling material) during the treatment of an infected root canal (The Japanese Journal of Conservative Dentistry, 34 (3),986-992,1991; and International Endodontic Journal, 24, 119-125, 1991).

A root canal therapeutic agent containing calcium hydroxide is believed to give less irritation to tissue and to have bactericidal activity due to strong alkalinity, soft tissue dissolving activity, analgesic activity, healing promoting activity, exudate stop activity, dental root absorption inhibiting activity, hard tissue formation inducing activity, and the like. As described above, calcium hydroxide is not merely an antiseptic but is believed to be a medicinal agent improving the healing of a root canal-periapical tissue.

A root canal filling material containing calcium hydroxide, which has excellent bactericidal activity and an activity to promote the healing of a root canal-periapical tissue, is useful, but insufficiently provides the activity to promote the healing of intractable pulpitis or apical periodontitis, for example, due to bacterial infection that is difficult to remove or to healing prevention by bacteria invaded into the lesion around a root canal or lesion at a root apex. There is therefore a demand for a root canal filling material capable of promoting the healing of intractable pulpitis or apical periodontitis.

### Citation List

### Patent Literature

Patent Document 1: JP 3473879 B
Patent Document 2: JP 4614376 B
Patent Document 3: JP 5827086 B

### Summary of Invention

### Technical Problem

The present invention has an objective to provide a root canal filling material composition having higher healing promoting activity than conventional root canal filling material compositions.

### Solution to Problem

The inventors of the present invention have repeated studies in order to solve the above problem and have found that a root canal filling material composition comprising a lithium salt promotes the differentiation from dental pulp stem cells to osteoblasts to activate or stimulate the bone metabolism in a periapical lesion and activates or stimulates immune response. The inventors of the present invention have further found that the root canal filling material composition consequently has an excellent activity to promote the healing of a root canal-periapical tissue and effectively promotes the healing of intractable pulpitis and apical periodontitis that cannot be healed by conventional root canal filling materials.

The present invention has been completed on the basis of the above findings and provides the following root canal filling material compositions.
[1] A root canal filling material composition comprising a lithium salt.
[2] The root canal filling material composition according to [1], wherein a concentration of the lithium salt is 0.0001 to 20% by weight relative to a total amount of the composition.
[3] The root canal filling material composition according to [1] or [2], further comprising a calcium compound.
[4] The root canal filling material composition according to [3], wherein a concentration of the calcium compound is 2 to 70% by weight relative to a total amount of the composition.
[5] The root canal filling material composition according to any one of [1] to [4], further comprising water.
[6] The root canal filling material composition according to [5], wherein the water is contained 1 to 2 times the lithium salt in terms of weight ratio.
[7] The root canal filling material composition according to any one of [1] to [6], further comprising a polyhydric alcohol.
[8] The root canal filling material composition according to [7], wherein a concentration of the polyhydric alcohol is 1 to 90% by weight relative to a total amount of the composition.
[9] The root canal filling material composition according to any one of [1] to [8], further comprising aluminum oxide ultrafine particles having a particle size of 1 nm to 1 µm and/or titanium oxide ultrafine particles having a particle size of 1 nm to 1 µm.
[10] The root canal filling material composition according to [9], wherein a total content of the aluminum oxide ultrafine particles and/or the titanium oxide ultrafine particles is 0.1 to 20% by weight relative to a total amount of the composition.
[11] The root canal filling material composition according to [1] or [2], wherein the root canal filling material composition is a combination of a paste A containing rosin and a paste B containing magnesium oxide and a vegetable oil, and one or both of the paste A and the paste B comprise the lithium salt.
[12] The root canal filling material composition according to [1] or [2], wherein the root canal filling material composition is a combination of a paste A and a paste B and hardens by mixing the paste A and the paste B, one or both of the paste A and the paste B comprise the lithium salt, one or both of the paste A and the paste B comprise a glass powder, the glass powder contains calcium oxide and silicon dioxide in a total amount of 50 to 100% by weight relative to a total amount of the glass powder, and in the glass powder, a weight ratio of the calcium oxide to the silicon dioxide (calcium oxide: silicon dioxide) is 6:4 to 3:7.
[13] The root canal filling material composition according to [12], wherein a content of the glass powder is 20% by weight or more relative to a total amount of the paste A and the paste B and is 70% by weight or less relative to a total amount of each of the paste A and the paste B.
[14] The root canal filling material composition according to [12] or [13], wherein the paste A further comprises at least one component selected from the group consisting of eugenol, guaiacol, a higher fatty acid, polyacrylic acid or a salt thereof, and phosphoric acid, and the paste B further comprises at least one component selected from the group consisting of calcium oxide, magnesium oxide, strontium oxide, zinc oxide, calcium phosphate, and aluminosilicate glass.
[15] The root canal filling material composition according to any one of [1] to [14] for use in immunostimulation in a lesion in a root canal and/or periapical tissue.
[16] The root canal filling material composition according to any one of [1] to [14] for use in differentiation induction from dental pulp stem cells to osteoblasts in a lesion in a root canal and/or periapical tissue.
[17] Use of a lithium salt for production of a root canal filling material composition.
[18] Use of a composition containing a lithium salt for production of a root canal filling material.
[19] Use of a lithium salt for a root canal filling material composition.
[20] Use of a composition containing a lithium salt for a root canal filling material.
[21] A method of treating an infected root canal and/or an infected periapical tissue, the method comprising filling a root canal of a patient having an infected root canal and/or an infected periapical tissue, with a lithium salt in an effective amount to treat the infected root canal and/or the infected periapical tissue.
[22] A method of treating an infected root canal and/or an infected periapical tissue, the method comprising filling a root canal of a patient having an infected root canal and/or an infected periapical tissue, with a composition containing a lithium salt in an effective amount to treat the infected root canal and/or the infected periapical tissue.
[23] Use of a lithium salt for production of an immunostimulant in a lesion in a root canal and/or periapical tissue.
[24] Use of a composition containing a lithium salt for production of an immunostimulant in a lesion in a root canal and/or periapical tissue.
[25] Use of a lithium salt for an immunostimulant in a lesion in a root canal and/or periapical tissue.
[26] Use of a composition containing a lithium salt for an immunostimulant in a lesion in a root canal and/or periapical tissue.
[27] A lithium salt for use in immunostimulation in a lesion in a root canal and/or periapical tissue.
[28] An immunostimulation method in a lesion in a root canal and/or periapical tissue, the method comprising filling a root canal of a patient having a lesion in a root canal and/or periapical tissue, with a lithium salt in an effective amount for immunostimulation in the lesion in the root canal and/or the periapical tissue.
[29] An immunostimulation method in a lesion in a root canal and/or periapical tissue, the method comprising filling a root canal of a patient having a lesion in a root canal and/or periapical tissue, with a composition containing a lithium salt in an effective amount for immunostimulation in the lesion in the root canal and/or the periapical tissue.
[30] Use of a lithium salt for production of a differentiation inducer from dental pulp stem cells to osteoblasts in a lesion in a root canal and/or periapical tissue.
[31] Use of a composition containing a lithium salt for production of a differentiation inducer from dental pulp stem cells to osteoblasts in a lesion in a root canal and/or periapical tissue.
[32] Use of a lithium salt for a differentiation inducer from dental pulp stem cells to osteoblasts in a lesion in a root canal and/or periapical tissue.
[33] Use of a composition containing a lithium salt for a differentiation inducer from dental pulp stem cells to osteoblasts in a lesion in a root canal and/or periapical tissue.
[34] A lithium salt for use in differentiation induction from dental pulp stem cells to osteoblasts in a lesion in a root canal and/or periapical tissue.
[35] A method of inducing differenciation from dental pulp stem cells to osteoblasts in a lesion in a root canal and/or periapical tissue, the method comprising filling a root canal of a patient having a lesion in a root canal and/or periapical tissue, with a lithium salt in an effective amount to induce differentiation from dental pulp stem cells to osteoblasts in the lesion in the root canal and/or the periapical tissue.
[36] A method of inducing differentiation from dental pulp stem cells to osteoblasts in a lesion in a root canal and/or periapical tissue, the method comprising filling a root canal of a patient having a lesion in a root canal and/or periapical tissue, with a composition containing a lithium salt in an effective amount to induce differentiation from dental pulp stem cells to osteoblasts in the lesion in the root canal and/or the tissue around the root apex.

### Advantageous Effects of Invention

Intractable pulpitis or apical periodontitis, for example, due to bacterial infection that is difficult to remove from the dental pulp or to healing prevention by bacteria invaded into the lesion around a root canal or at a root apex is difficult to heal even with a root canal filling material containing calcium hydroxide that is believed to have healing promoting activity.

In contrast, the root canal filling material composition of the present invention comprises a lithium salt, thus promotes or induces differentiation from dental pulp stem cells to osteoblasts, and increases white blood cells to stimulate immunity. On the basis of these activities, the root canal filling material composition of the present invention suppresses the inflammation of a lesion such as a pustule in a dental pulp or around a root apex even in intractable pulpitis or apical periodontitis, and effectively promotes healing.

When the root canal filling material composition of the present invention comprises calcium hydroxide in addition to the lithium salt, the composition has excellent bactericidal activity and anti-inflammatory activity derived from calcium hydroxide, and the activity of promoting the healing of a root canal-root apex periodontal tissue is markedly or synergistically improved.

The inventors of the present invention have found that when a root canal filling material composition comprising a lithium salt is filled in a root canal, the lithium salt is insufficiently eluted in some cases. In this case, a larger amount of the lithium salt is required to be contained, and this may increase the price or may limit the preparation.

In contrast, a root canal filling material composition comprising water and a polyhydric alcohol in addition to the lithium salt and calcium hydroxide easily elutes the lithium salt after being filled in a root canal, and the root canal filling material composition comprising a small amount of a lithium salt can provide the function of healing a root canal-periapical tissue.

When a root canal filling material composition comprising calcium hydroxide is stored, calcium hydroxide may precipitate and separate, and when used, the composition may mainly elute only the liquid components, or calcium hydroxide may solidify in a syringe, and the paste may be difficult to discharge. In contrast, the root canal filling material composition comprising a lithium salt, calcium hydroxide, water, and a polyhydric alcohol can suppress such solid-liquid separation during storage.

The inventors of the present invention have also found that when calcium hydroxide is added to a root canal filling material composition comprising a lithium salt, the paste after preparation may harden or dry, and such a preparation is difficult to catch on a lentulo or to smoothly discharge from a syringe container or the like and is difficult to apply. In contrast, when the root canal filling material composition of the present invention is a composition comprising a lithium salt, calcium hydroxide, water, and a polyhydric alcohol, and the water is contained 1 to 2 times the lithium salt in terms of weight ratio, the composition has an appropriate flow, and the paste does not harden and is unlikely to dry. Hence, the composition can be smoothly discharged from a syringe container or the like and has excellent operability. The composition is unlikely to dry, thus the container or package of the composition needs no special design, and the composition can be easily developed to a product.

As mentioned above, according to the studies by the inventors of the present invention, a root canal filling material composition comprising a lithium salt may insufficiently elute the lithium salt after the root canal filling material composition is filled in a root canal. When a two-paste type root canal filling material composition comprises a paste A containing a higher fatty acid and rosin and a paste B containing magnesium oxide and a vegetable oil in combination, and one or both of the paste A and the paste B contain a lithium salt, the lithium salt is easily eluted after a mixture of the paste A and the paste B is filled in a root canal, and the root canal filling material composition comprising a small amount of the lithium salt can provide an excellent promoting function of healing a root canal-periapical tissue.

When a root canal filling material composition comprises a paste A containing a higher fatty acid and rosin and a paste B containing magnesium oxide and a vegetable oil in combination, and one or both of the paste A and the paste B contain a lithium salt, the root canal filling material composition has an appropriate flow and thus has high operability. For example, the composition is easily filled in a root canal and is easily caught on an instrument such as a lentulo and a gutta-percha point. The excellent operability includes high stringing property.

If the setting time is excessively short, the paste sets during mixing, and the hardened paste is difficult to feed into a root canal. If the setting time is excessively long, contamination is caused in a root apex with an exudate or blood from the periphery and reduces the sealing performance of the root apex. The combination composition has an appropriate setting time.

The combination composition has satisfactory storage stability, and the solid-liquid separation during storage is suppressed.

When a root canal filling material composition comprises a paste A and a paste B in combination and hardens by mixing the paste A and the paste B, one or both of the paste A and the paste B comprise a glass powder, the glass powder contains calcium oxide and silicon dioxide in a total amount of 50 to 100% by weight relative to the total amount of the glass powder, the weight ratio of the calcium oxide to the silicon dioxide (calcium oxide: silicon dioxide) is 6: 4 to 3:7 in the glass powder, and one or both of the paste A and the paste B comprise a lithium salt, the lithium salt is easily eluted after a mixture of the paste A and the paste B is filled in a root canal, and the root canal filling material composition comprising a small amount of a lithium salt can provide a promoting function of healing a root canal-root apex periodontal tissue.

The root canal filling material composition comprising a glass powder and a lithium salt has an appropriate flow and thus has high operability. For example, the composition is easily filled in a root canal and is easily caught on an instrument such as a lentulo and a gutta-percha point. The excellent operability includes high stringing property. The composition also has an appropriate setting time. The composition has satisfactory storage stability, and the solid-liquid separation during storage is suppressed.

The root canal filling material composition comprising a glass powder and a lithium salt forms, in the living body, a crystal phase belonging to a space group of P6_{3/}m, such as hydroxyapatite, or forms a bone-like crystal. Bones and teeth, which constitute a large part of the hard tissue in the living body, contains hydroxyapatite as a main component. The root canal filling material composition generates, from its surface, a bone-like crystal having the same structure as the main component of teeth, then fills a small void formed between the filler and a dentine, and can be integrated through the crystal with the dentine and bone tissue. In particular, the property has a beneficial effect on filling a tooth root canal that is difficult to seal, such as a tooth with no root apex, including a deciduous tooth and an immature permanent tooth, a tooth without formation of an apical seat in infected root canal treatment, and a tooth having a root apex that is not widely opened by apicoectomy. The composition accordingly provides the close contact with a dentine and bone tissue to result in a satisfactory sealing performance and induces a hard tissue even in a case in which complete sealing cannot be achieved clinically.

When in the root canal filling material composition comprising a glass powder and a lithium salt, the paste A comprises, as components giving a setting property, one or more components selected from eugenol, guaiacol, a higher fatty acid, polyacrylic acid, and phosphoric acid, and the paste B comprises, as components giving a setting property, one or more components selected from calcium oxide, magnesium oxide, strontium oxide, zinc oxide, calcium phosphate, and aluminosilicate glass, the flow, operability, setting time, storage stability, and formation inducibility of bone-like crystals are further improved.

### Brief Description of Drawings

Figs. 1 are each an X-ray micro-CT image of the left mandibular first molar 4 weeks after filling the root canal filling material composition in Example 18 or Comparative Example 1 inside the apical foramen with lesion of a rat.
Fig. 2 is a graph showing the time course of lesion volume after filling each root canal filling material composition in Example 5 and Comparative Example 1 inside the apical foramen with lesion of a rat.
Fig. 3 is a graph showing time course of lesion volume after filling each root canal filling material composition in Example 3 and Comparative Example 1 inside the apical foramen with lesion of a rat.
Fig. 4 is a graph showing time course of lesion volume after filling each root canal filling material composition in Examples 1 to 3 and Comparative Example 1 inside the apical foramen with lesion of a rat.
Figs. 5 are histopathological section images prepared as follows: each root canal filling material composition in Example 18 and Comparative Example 1 was filled inside the apical foramen with lesion of a mouse; and after 4 weeks, a tissue section of the periapical lesion was prepared and was subjected to in-situ hybridization. The arrows indicate Colla1 or Runx2 positive cells.
Figs. 6 are images prepared as follows: each root canal filling material composition in Example 18 and Comparative Example 1 was filled inside the apical foramen with lesion of a mouse; and after 4 weeks, a tissue section of the periapical lesion was subjected to immunofluorescence staining with a cell surface marker on B cells.

### Description of Embodiments

### The present invention will now be described in detail. Root canal filling material composition of present invention

A root canal filling material composition of the present invention is the root canal filling material composition that comprises a lithium salt.

The root canal filling material is also called a sealer for root canal filling, an intracanal medicament, a cavity filler, or a perforation repair material, for example.

### (Lithium salt)

The lithium salt may be any of inorganic acid salts and organic acid salts. Examples of the inorganic acid salt include a hydrochloride, a carbonate, a sulfate, a phosphate, a hydrobromide, and a nitrate. Examples of the organic acid salt include a polycarboxylate such as an orotinate (also called "orotate" or "uracil 6-carboxylate"), a citrate, a fumarate, a maleate, a succinate, and a malonate, a lactate, a tartrate, an acetate, a butyrate, a palmitate, a stearate, a methanesulfonate, a toluenesulfonate, a tosylate, and a napadisilate.

Specifically, an inorganic acid salt is preferred, and a hydrochloride and a carbonate are more preferred.

Lithium salts may be used singly or in combination of two or more of them.

The concentration of the lithium salt can be, for example, 0.0001% by weight or more, 0.001% by weight or more, or 0.005% by weight or more and is preferably 0.01% by weight or more, more preferably 0.05% by weight or more, and even more preferably 0.1% by weight or more relative to the total amount of the composition. A composition containing the lithium salt at a concentration within the above range can sufficiently promote the healing of pulpitis or apical periodontitis. The concentration of the lithium salt is preferably 20% by weight or less, more preferably 15% by weight or less, even more preferably 12% by weight or less, still more preferably 10% by weight or less, and further preferably 6% by weight or less relative to the total amount of the composition. A composition containing the lithium salt at a concentration within the above range does not cause lithium poisoning such as anorexia and emesis.

### (Use or Application)

When the root canal filling material composition of the present invention is filled in the lesion in a root canal or periapical tissue, the composition induces the differentiation from dental pulp stem cells to osteoblasts and increases white blood cells to activate immunity. The root canal filling material composition of the present invention therefore can be used as a composition for inducing the differentiation of osteoblasts (specifically, inducing the differentiation of osteoblasts in the lesion in a root canal and/or periapical tissue), a composition for increasing white blood cells (specifically, increasing white blood cells in the lesion in a root apex and/or periapical tissue), and a composition for immunostimulation (specifically, immunostimulation in the lesion in a root apex and/or periapical tissue.

The differentiation of osteoblasts with the root canal filling material composition of the present invention is induced by the promotion of Colla1 expression or the promotion of Runx2 expression, and thus the root canal filling material composition of the present invention can be used as a composition for promoting Colla1 expression (specifically, a composition for promoting Colla1 expression in the lesion in a root canal and/or periapical tissue) or as a composition for promoting Runx2 expression (specifically, a composition for promoting Runx2 expression in the lesion in a root canal and/or periapical tissue).

As described above, pulpitis and apical periodontitis involve bacterial infection. Hence, the present invention encompasses a method of treating an infected root canal and/or an infected periapical tissue, and the method comprises filling the root canal of a patient having an infected root canal and/or an infected periapical tissue, with a composition comprising a lithium salt in an amount sufficient to treat the infected root canal and/or the infected periapical tissue.

The present invention also encompasses an immunostimulation method in the lesion in a root canal and/or periapical tissue, and the method comprises filling a root canal of a patient having a lesion in the root canal and/or the periapical tissue, with a composition comprising a lithium salt in an amount sufficient for immunostimulation in the lesion in the root canal and/or the periapical tissue.

The present invention also encompasses a method of inducing differentiation from dental pulp stem cells to osteoblasts in the lesion in a root canal and/or periapical tissue, and the method comprises filling a root canal of a patient having a lesion in the root canal and/or the periapical tissue, with a composition containing a lithium salt in an amount sufficient to induce the differentiation from dental pulp stem cells to osteoblasts in the lesion in the root canal and/or the periapical tissue.

The dosage amount or the filling amount of the composition containing a lithium salt sufficient to treat an infected root canal and/or an infected periapical tissue depends on the type of tooth or the shape of a root canal, and about 0.01 to 0.03 mL of a composition containing a lithium salt at the above concentration can be used. The dosage amount or the filling amount of the composition containing a lithium salt sufficient for immunostimulation or for inducing the differentiation from dental pulp stem cells to osteoblasts, in a lesion in a root canal and/or periapical tissue is substantially the same as above.

The patient can be a non-human animal or a human and is particularly preferably a human.

### (Elution properties of lithium salt)

The root canal filling material composition of the present invention preferably has an absolute daily lithium amount in immersion liquid of 0.02 µg or more, more preferably 0.05 µg or more, and even more preferably 0.1 µg or more, as determined in accordance with the method in "Lithium elution test from apical foramen" in EXAMPLES. The amount can be 1 µg or more. The amount is preferably 5 mg or less, more preferably 1 mg or less, and even more preferably 0.1 mg or less. The amount can be 10 µg or less.

### First embodiment of root canal filling material composition of present invention

### (Calcium compound)

In a first embodiment, the root canal filling material composition of the present invention can further comprise a calcium compound.

Examples of the calcium compound include calcium hydroxide, calcium carbonate, calcium sulfate, calcium chloride, calcium acetate, calcium oxide, tricalcium phosphate, calcium monohydrogen phosphate, calcium dihydrogen phosphate, and calcium dihydrogen pyrophosphate. Specifically, calcium hydroxide and calcium carbonate are preferred, and calcium hydroxide is more preferred.

Calcium compounds may be used singly or in combination of two or more of them.

In the composition containing a calcium compound (particularly calcium hydroxide), the concentration of the calcium compound (particularly calcium hydroxide) is preferably 2% by weight or more, more preferably 5% by weight or more, even more preferably 10% by weight or more, and still more preferably 20% by weight or more relative to the total amount of the composition. When containing calcium hydroxide at a concentration within the above range, the composition sufficiently provides the pharmaceutical effect, particularly the pharmaceutical effect of calcium hydroxide. The concentration of the calcium compound (particularly calcium hydroxide) is preferably 70% by weight or less, more preferably 50% by weight or less, and even more preferably 40% by weight or less relative to the total amount of the composition. When containing the calcium compound at a concentration within the above range, the composition is moderately hard and can be easily discharged even from a syringe container.

### (Water)

The root canal filling material composition of the present invention in the first embodiment may further comprise water.

In the composition containing water, the concentration of water is preferably 1 time or more the concentration (% by weight) of the lithium salt, more preferably 1.1 times or more, even more preferably 1.2 times or more, and still more preferably 1.3 times or more. When containing water at a concentration within the above range, the paste is not hard. The concentration of the water is preferably 2 times or less the concentration (% by weight) of the lithium salt, more preferably 1.8 times or less, even more preferably 1.7 times or less, and still more preferably 1.6 times or less. A composition containing water at an excessively high proportion easily dries, but a composition containing water at a proportion within the above range is prevented from drying during storage.

The concentration of water is preferably 0.001% by weight or more, more preferably 0.005% by weight or more, even more preferably 0.01% by weight or more, still more preferably 0.1% by weight or more, and further preferably 1% by weight or more relative to the total amount of the composition. The concentration is preferably 70% by weight or less, more preferably 50% by weight or less, even more preferably 40% by weight or less, still more preferably 30% by weight or less, and further preferably 25% by weight or less.

### (Polyhydric alcohol)

The root canal filling material composition of the present invention in the first embodiment may further comprise a polyhydric alcohol.

The polyhydric alcohol is added in order to make a calcium compound into a paste-like preparation. Examples of the polyhydric alcohol include dihydric alcohols such as propylene glycol, polypropylene glycol, and polyethylene glycol and trihydric alcohols such as glycerol. Specifically, propylene glycol, polyethylene glycol, and glycerol are preferred.

The polypropylene glycol preferably has a polymerization degree of 100 to 1,000, more preferably 200 to 600, and even more preferably 300 to 400.

The polyethylene glycol preferably has a polymerization degree of 100 to 1,000, more preferably 200 to 600, and even more preferably 300 to 400.

Polyhydric alcohols may be used singly or in combination of two or more of them. "Two or more of them" includes, in addition to a case in which two or more polyhydric alcohols different in compound type are contained, a case in which two or more polypropylene glycols different in polymerization degree are contained and a case in which two or more polyethylene glycols different in polymerization degree are contained, for example.

In the composition containing a polyhydric alcohol, the concentration of the polyhydric alcohol is preferably 1% by weight or more, more preferably 5% by weight or more, even more preferably 10% by weight or more, still more preferably 20% by weight or more, and further preferably 30% by weight or more relative to the total amount of the composition. The concentration of the polyhydric alcohol is preferably 90% by weight or less, more preferably 70% by weight or less, even more preferably 60% by weight or less, and still more preferably 50% by weight or less relative to the total amount of the composition. A composition containing the polyhydric alcohol at a concentration within the above range can be made into an easy-handling viscous paste preparation.

### (Ultrafine particles of aluminum oxide/titanium oxide)

The root canal filling material composition of the present invention in the first embodiment may further comprise aluminum oxide ultrafine particles having a particle size of 1 nm to 1 µm and/or titanium oxide ultrafine particles having a particle size of 1 nm to 1 µm.

When stored in a container, the root canal filling material composition comprising a calcium compound (particularly calcium hydroxide) is likely to separate into the calcium compound (particularly calcium hydroxide) and a liquid component and is likely to be difficult to push out from the container such as a syringe container. When the composition of the present invention comprises the above aluminum oxide ultrafine particles and/or titanium oxide ultrafine particles, the solid-liquid separation during storage of the composition is suppressed.

The ultrafine particles have a particle size of 1 nm or more and can have a particle size of 5 nm or more, or 10 nm or more. The ultrafine particles have a particle size of 1 µm or less and can have a particle size of 500 nm or less, 300 nm or less, 50 nm or less, or 20 nm or less. When the ultrafine particles have a particle size within the above range, the separation between the calcium compound (particularly calcium hydroxide) and the liquid component can be sufficiently suppressed.

In the present invention, the particle size of ultrafine particles is the average particle size of primary particles and is determined by observation under a transmission electron microscope.

In the composition containing aluminum oxide ultrafine particles and/or titanium oxide ultrafine particles, the total concentration of the ultrafine particles can be, for example, 0.1% by weight or more and is preferably 0.5% by weight or more, more preferably 1% by weight or more, and even more preferably 2% by weight or more relative to the total amount of the composition. When the ultrafine particles are contained at a concentration within the above range, the thickening effect is sufficiently achieved, and the solid-liquid separation is suppressed. The concentration of the ultrafine particles can be, for example, 20% by weight or less and is preferably 10% by weight or less, more preferably 5% by weight or less, and even more preferably 4% by weight or less relative to the total amount of the composition. When containing the ultrafine particles at a concentration within the above range, the composition is moderately hard and is easily discharged from a syringe container.

### (Additional components)

The root canal filling material composition of the present invention in the first embodiment may further comprise an ointment base such as liquid paraffin, petrolatum, oil (a vegetable oil such as olive oil and peanut oil), wax, resin, and syrup and a thickener such as cellulose and ester gum in order to control the flow of a paste. When the composition comprises an ointment base, a surfactant is preferably used in combination.

The root canal filling material composition of the present invention in the first embodiment may comprise an X-ray contrast medium or radiopaque medium such as barium sulfate, zirconium oxide, bismuth subnitrate, bismuth trioxide, bismuth carbonate, zinc oxide, and iodoform. The X-ray contrast or opacity required for a root canal filling material composition is required to be an "aluminum plate having a thickness of 3 mm or more" according to JIS-T6522 [Dental root canal sealing materials] and varies with the type of used X-ray contrast medium or radiopaque medium. For example, barium sulfate is contained at a content of 10% by weight or more relative to the total amount of the composition.

In addition, the composition can comprise a component usable in a root canal filling material, such as an antimicrobial agent, as long as the effect of the invention is not impaired.

Additional components (components other than the lithium salt, the calcium compound, water, the polyhydric alcohol, the ultrafine particles of aluminum oxide, and the ultrafine particles of titanium oxide) may be used singly or in combination of two or more of them.

### (Flow)

The root canal filling material composition of the present invention in the first embodiment preferably has a flow (spreading average) of 25 to 50 mm and more preferably 25 to 40 mm, where the flow is determined as follows: 75 µL of a composition is interposed between glass plates; a weight load of 2.5 kg is applied thereon for 7 minutes; and the maximum distance and the minimum distance between parallel tangents contacting to the spread paste are measured. When the flow is within the above range, the solid-liquid separation during storage of the composition is more effectively suppressed, and the composition has an appropriate flowability for application.

### (Preparation method)

The root canal filling material composition of the present invention in the first embodiment can be prepared by mixing and kneading liquid components (for example, including water and a polyhydric alcohol) and solid components (including a lithium salt and, for example, a calcium compound).

### (Usage)

The root canal filling material composition of the present invention in the first embodiment, for example, can be loaded in a syringe container, then an 18 to 25-gauge blunt needle can be attached to the syringe container, and the composition can be filled in a root canal.

To remove the composition from a root canal, 5 to 20% by weight (specifically about 10% by weight) of hypochlorous acid and 1 to 5% by weight (specifically about 3% by weight) of hydrogen peroxide water can be alternately used to wash the composition, or ultrasonic cleaning using purified water and an ultrasonic tip can be performed to remove the composition. When the root canal filling material composition of the present invention comprises aluminum oxide ultrafine particles and/or titanium oxide ultrafine particles, the paste does not harden for several days to several weeks after filling and thus can be easily removed.

### Root canal filling material composition of present invention in second embodiment

In a second embodiment, the root canal filling material composition of the present invention is a combination composition of a paste A containing a higher fatty acid and rosin and a paste B containing magnesium oxide and a vegetable oil, and one or both of the paste A and the paste B comprise a lithium salt. The composition is used after mixing the paste A and the paste B.

### (Lithium salt)

The lithium salt is contained in one or both of the paste A and the paste B. The lithium salt is preferably contained only in the paste B, and this can prevent setting by the progress of saponification reaction with a higher fatty acid or rosin in the paste A.

As mentioned above, the concentration of the lithium salt is preferably 0.0001 to 20% by weight relative to the total amount of the composition, and in the second embodiment, the "total amount of the composition" is the total amount after mixing the paste A and the paste B.

### (Higher fatty acid)

The higher fatty acid in the paste A undergoes saponification reaction when coming into contact with magnesium oxide in the paste B, and consequently, the mixed paste as a sealer hardens. The higher fatty acid preferably has a carbon number of 15 or more not to emit irritating smell and is preferably liquid around room temperature because the paste A is easily made into a paste. The higher fatty acid may be any of saturated fatty acids and unsaturated fatty acids. A higher fatty acid that has a carbon number of 15 or more and is liquid around room temperature is more preferred. Of the saturated fatty acids, a saturated fatty acid having a branched carbon chain, such as isostearic acid, is particularly preferred. Of the unsaturated fatty acids, linoleic acid, linolenic acid, arachidonic acid, clupanodonic acid, palmitoleic acid, and the like can be particularly preferably used.

The content of the higher fatty acid can be 20 to 50% by mass and is preferably 30 to 40% by mass relative to the total amount of the paste A. If the higher fatty acid is contained at an excessively low content, the setting time is prolonged, and an exudate or blood outside an apical foramen is likely to contaminate the inner wall of the root canal, or sealing performance at a root apex is likely to deteriorate. In addition, a decrease of the liquid component increases the viscosity of the paste A itself, and the paste has an excessively high flow when used and is difficult to use. If the higher fatty acid is contained at an excessively high content, the setting time is excessively short, thus the paste solidifies during root canal filling operation, and the paste is difficult to feed into a root canal, or the operation margin time is likely to shorten.

### (Rosin)

The rosin in the paste A is a solid resin prepared by removing essential oils from a secreted material of various plants belonging to the genus Pinus and is not specifically limited, and any rosin conventionally used in the dental field can be used. The rosin is typically composed of 90% of resin acids and 10% of neutral substances. The resin acids include, as the main components (about 90%), abietic acid isomerized by heat, and the rosin contains pimaric acid and isopimaric acid as other components and contains almost no essential oils. A rosin from France contains pimaric acid at a high content. Rosins prepared by hydrogenation of unsaturated bond moieties of these rosins are also included.

The rosin also undergoes saponification reaction when coming into contact with magnesium oxide, and this promotes setting reaction of mixed pastes.

The content of the rosin can be 40 to 80% by mass and is preferably 50 to 70% by mass relative to the total amount of the paste A. If the rosin is contained at an excessively low content, the setting time is prolonged, and an exudate or blood outside an apical foramen may cause contamination to reduce the sealing performance at the root apex. If the rosin is contained at an excessively high content, the paste has an excessively high flow when used and is likely to be difficult to feed into a root canal.

### (Magnesium oxide)

The magnesium oxide in the paste B instantly undergoes saponification reaction when coming into contact with the higher fatty acid and the rosin, and the mixture hardens. The setting time is thus affected by the content of magnesium oxide. If magnesium oxide is contained at an excessively low content, the setting reaction becomes slow. If magnesium oxide is contained at an excessively high content, the setting reaction becomes fast, and the mixture is difficult to fill in a root canal. The content of magnesium oxide suitable for the optimum setting time (about 10 to 30 minutes) varies with the types or contents of other components contained in the composition and is, for example, 5 to 30% by mass and specifically preferably 10 to 20% by mass relative to the total amount of the paste B.

### (Vegetable oil)

The vegetable oil in the paste B may be any natural component to be the base material in the paste, and examples include vegetable oils such as olive oil, peanut oil, rapeseed oil, soybean oil, safflower oil, cottonseed oil, corn oil, and evening primrose oil. An ester compound of glycerol and a higher fatty acid such as linoleic acid, oleic acid, linolenic acid, palmitic acid, and isostearic acid is also contained in a vegetable oil at a high content and thus is also included in the vegetable oil in the present invention.

A paste prepared by mixing the paste A and the paste B preferably has a moderately high flow and has such a flow that the paste does not flow in a root canal. If the vegetable oil is contained at an excessively low content, the paste has an excessively high flow when used, and the paste is difficult to feed into a root canal, or the paste B is difficult to prepare. If the vegetable oil is contained at an excessively high content, the composition has an excessively low flow when used and is difficult to handle. The content of the vegetable oil for satisfying an ideal paste flow can be 10 to 40% by mass and is specifically preferably 15 to 30% by mass relative to the total amount of the paste B.

### (Additional components)

Additional components that can be contained in the root canal filling material composition of the present invention in the second embodiment are substantially the same as in the first embodiment. In addition to the "additional components" listed in the first embodiment, polyhydric alcohols such as propylene glycol, polypropylene glycol, polyethylene glycol, and glycerol can be contained as a thickener. Water can also be contained.

The X-ray contrast medium or radiopaque medium can be contained in one or both of the paste A and the paste B and is preferably contained in the paste B in order to prevent the solid-liquid separation of the composition. As for the content of the X-ray contrast medium or radiopaque medium, for example, the concentration of barium sulfate can be 10% by weight or more relative to the total amount of the composition after mixing the paste A and the paste B.

When an ointment base or a thickener is used in order to control the viscosity of the paste A and the paste B, the ointment base or thickener can be contained in one or both of the paste A and the paste B. The vegetable oil is contained in the paste B and can also be contained in the paste A in order to control the viscosity.

In the root canal filling material composition of the present invention in the second embodiment, in addition to the "additional components" in the first embodiment, one or both of the paste A and the paste B can contain silicon dioxide ultrafine particles having a particle size of 1 nm to 1 µm in order to control the viscosity.

When the ultrafine particles are contained, the content can be 0.1% by weight or more and is preferably 1% by weight or more and more preferably 2% by weight or more relative to each total amount of the paste A and the paste B. The content is preferably 10% by weight or less, more preferably 8% by weight or less, and even more preferably 5% by weight or less relative to each total amount of the paste A and the paste B. When the content is within the above range, the thickening effect is sufficiently achieved, and the solid-liquid separation is suppressed.

The root canal filling material composition of the present invention in the second embodiment can contain eugenol but preferably contains no eugenol.

The root canal filling material composition of the present invention in the second embodiment preferably contains no calcium oxide, calcium hydroxide, or magnesium hydroxide. If such a component is contained, saponification (setting) reaction with a fatty acid or rosin immediately proceeds, thus setting starts upon mixing the pastes, and mixing operation or filling operation is likely to be difficult. If such a component is contained in the paste B, saponification reaction with a vegetable oil proceeds, and the paste B may solidify (set) in a container such as a syringe during storage.

In the present invention, "containing no" certain component includes a case in which an unavoidable amount of the component is contained.

In the root canal filling material composition of the present invention in the second embodiment, the combination weight ratio of the paste A to the paste B (paste A:paste B) is preferably 1:0.5 to 1.5, more preferably 1:0.8 to 1.2, and particularly preferably 1:1.

### (Preparation method)

Each of the paste A and the paste B of the root canal filling material composition of the present invention in the second embodiment can be prepared by mixing and kneading liquid components and solid components.

### (Container)

The paste A and the paste B may be stored in separate containers or may be separately stored in the respective storage parts of a one-pack type container having two storage parts.

Examples of the one-pack type container having two storage parts include containers each having two rooms. In a container, the two rooms are separated by a partition, and the paste A and the paste B are filled in the respective rooms and are mixed by breaking the partition when used. In another container, each storage part has an ejection port or a portion to be an ejection port, and the paste A and the paste B are discharged from the respective ejection ports and are mixed when used. Examples of the latter container include a dual-syringe container from which the loaded paste A and paste B can be taken at an intended ratio by a single push and a dual-syringe container having a mixing tip at an ejection port from which a mixture of the paste A and the paste B at an intended ratio can be filled in a root canal by a single push.

### (Usage)

According to the root canal filling material composition of the present invention in the second embodiment, the paste A and the paste B can bemixed and be filled, for example, in a root canal. After filling, magnesium oxide undergoes saponification reaction with a higher fatty acid and rosin, and the composition hardens in about 5 minutes to 2 hours (preferably about 10 minutes to 30 minutes).

### Root canal filling material composition of present invention in third embodiment

In a third embodiment, the root canal filling material composition of the present invention is a root canal filling material composition that is a combination of a paste A and a paste B and hardens by mixing the paste A and the paste B. One or both of the paste A and the paste B comprise a lithium salt, and one or both of the paste A and the paste B comprise a glass powder. The glass powder contains calcium oxide and silicon dioxide in a total amount of 50 to 100% by weight relative to the total amount of the glass powder, and in the glass powder, the weight ratio of the calcium oxide to the silicon dioxide (calcium oxide: silicon dioxide) is 6:4 to 3:7.

### (Lithium salt)

The lithium salt is contained in one or both of the paste A and the paste B. Particularly when the paste A contains a setting property imparting component (specifically, a higher fatty acid, polyacrylic acid or a salt thereof, and/or phosphoric acid) as described later, the lithium salt is preferably contained only in the paste B in order to prevent setting by the progress of saponification reaction with a component in the paste A.

As mentioned above, the concentration of the lithium salt is preferably 0.0001 to 20% by weight relative to the total amount of the composition, and the "total amount of the composition" is the total amount after mixing the paste A and the paste B and is the total amount of the paste A and the paste B.

### (Glass powder)

In the root canal filling material composition of the present invention, one or both of the paste A and the paste B contain the powder glass having the above particular formula as a filler. A composition containing a larger amount of the powder glass has a higher performance of precipitating bone-like crystals and has a higher sealing performance. Hence, the content of the powder glass is preferably 20% by weight or more and can be 30% by weight or more or 40% by mass or more relative to the total amount of the paste A and the paste B.

A composition containing an excessively large amount of the powder glass may have a lower setting property or poor operability when used. Hence, the content of the powder glass is preferably 70% by weight or less and can be 60% by weight or less or 50% by weight or less relative to each paste either when the powder glass is contained in one of the paste A and the paste B or when the powder glass is contained in both the paste A and the paste B.

There have been many studies and patents in which a powder glass is mixed with an inorganic material such as bioceramics, a biodegradable polymer, an organic resin, or a similar material to improve the operability of a resulting composite or the strength of a hardened product, and the formation of apatite-like crystals from the glass surface in the living body and the bonding of the crystals with a bone or a tooth have been known. Such properties are called "bioactivity", and various glass formulations having the bioactivity have been examined and studied. As the constituent and formulation, calcium oxide and silicon dioxide are contained at a weight ratio of 6:4 to 3:7 in terms of calcium oxide:silicon dioxide, at 50 to 100% by mass. In addition, an alkali metal oxide can be contained at 0 to 40% by weight, and phosphoric acid can be contained at 0 to 10% by weight. An X-ray contrast component such as lanthanum oxide can also be contained.

The powder glass can be prepared in a usual manner or can be a commercially available product.

### (Setting property imparting component of paste A)

The paste A can comprise a component of imparting a setting property in order to promote setting by mixing the paste A and the paste B. Examples of the component include eugenol, guaiacol, a higher fatty acid, polyacrylic acid or a salt thereof, and phosphoric acid. Preferred examples of the higher fatty acid are the same as in the root canal filling material composition of the present invention in the second embodiment. Setting property imparting components may be used singly or in combination of two or more of them.

The content of the setting property imparting component is determined by the relation to the content of the powder glass contained as the filler. In other words, the content is required to be such a content as to harden the mixture of the pastes and to be a content suitable to allow the powder glass to provide a function of precipitating bone-like crystals and to maintain a satisfactory sealing performance. From this viewpoint, the content of the setting property imparting component is preferably 5% by weight or more, more preferably 10% by weight or more, even more preferably 20% by weight or more, and still more preferably 40% by weight or more relative to the total amount of the paste A. The paste A may contain no powder glass, and thus the paste A can contain the setting property imparting component at 100% by weight in the total amount of the paste A.

### (Setting property imparting component of paste B)

The paste B can comprise a component of imparting a setting property in order to promote setting by mixing the paste A and the paste B. Examples of the component include calcium oxide, magnesium oxide, strontium oxide, zinc oxide, calcium phosphate, and aluminosilicate glass. Setting property imparting components may be used singly or in combination of two or more of them.

The content of such a component is determined by the relation to the content of the powder glass contained as the filler in the paste B. The content of the setting property imparting component is preferably 5% by weight or more, more preferably 10% by weight or more, even more preferably 20% by weight or more, and still more preferably 40% by weight or more relative to the total amount of the paste B. The paste B may contain no powder glass, and thus the paste B can contain the setting property imparting component at 100% by weight in the total amount of the paste B.

The aluminosilicate glass differs from the above-described powder glass and has no function of forming bone-like crystals in the living body. The aluminosilicate glass, however, has been known in the dental field to be a hardenable material that hardens when mixed with polyacrylic acid or phosphoric acid, and can be selected as the component of imparting a setting property by mixing.

### (Additional components)

Additional components that can be contained in the root canal filling material composition of the present invention in the third embodiment are substantially the same as in the first embodiment. In addition to the "additional components" listed in the first embodiment, polyhydric alcohols such as propylene glycol, polypropylene glycol, polyethylene glycol, and glycerol can be contained as a thickener. Water can also be contained.

The X-ray contrast medium or radiopaque medium can be contained in one or both of the paste A and the paste B and is preferably contained in the paste B in order to prevent the solid-liquid separation of the composition. As for the content of the X-ray contrast medium or radiopaque medium, for example, the concentration of barium sulfate can be 10% by weight or more relative to the total amount of the composition after mixing the paste A and the paste B.

When an ointment base or a thickener is used in order to control the viscosity of the paste A and the paste B, the ointment base or thickener can be contained in one or both of the paste A and the paste B.

In the root canal filling material composition of the present invention in the third embodiment, in addition to the "additional components" in the first embodiment, one or both of the paste A and the paste B can contain silicon dioxide ultrafine particles having a particle size of 1 nm to 1 µm in order to control the viscosity.

When the ultrafine particles are contained, the content can be 0.1% by weight or more and is preferably 1% by weight or more and more preferably 2% by weight or more relative to each total amount of the paste A and the paste B. The content is preferably 10% by weight or less, more preferably 8% by weight or less, and even more preferably 5% by weight or less. When the content is within the above range, the thickening effect is sufficiently achieved, and the solid-liquid separation is suppressed.

In the root canal filling material composition of the present invention in the third embodiment, the combination weight ratio of the paste A to the paste B (paste A:paste B) is preferably 1:0.5 to 1.5, more preferably 1:0.8 to 1.2, and particularly preferably 1:1.

### (Preparation method)

Each of the paste A and the paste B of the root canal filling material composition of the present invention in the third embodiment can be prepared by mixing and kneading liquid components and solid components.

### (Container)

The container in which the paste A and the paste B are loaded is the same as in the second embodiment.

### (Usage)

According to the root canal filling material composition of the present invention in the third embodiment, the paste A and the paste B can be mixed and be filled, for example, in a root canal. After filling, the composition hardens in about 5 minutes to 3 hours.

The root canal filling material composition of the present invention comprises at least a lithium salt and can be any composition in addition to the first to third embodiments exemplified above.

### EXAMPLES

The present invention will next be described in further detail with reference to examples, but the present invention is not limited thereto.

### (1) Preparation of root canal filling material composition

As the first embodiment of the present invention, paste-like root canal filling material compositions each having a formula shown in Tables 1 to 4 were prepared by mixing a previously mixed liquid component and a previously mixed solid component and kneading the mixture in a porcelain mortar.

As the second and third embodiments of the present invention, pastes A each having a formula shown in Table 5 and pastes B each having a formula shown in Table 6 were prepared by kneading all the respective components in a porcelain mortar. The formulae of the powder glasses in Table 6 are shown in Table 7.

**Table 1**

| | | | | | | |
|---|---|---|---|---|---|---|
| (unit: % by weight) | | | | | | |

| | Lithium salt concentration | | | | | Without lithium salt |
|---|---|---|---|---|---|---|
| | Example 1 | Example 2 | Example 3 | Example 4 | Example 5 | Comparative Example 1 |
| Lithium carbonate | 0.01 | 0. 1 | 1. 0 | 6. 0 | 12.0 | - |
| Lithium chloride | - | - | - | - | - | |
| Purified water | 0.01 | 0. 1 | 1. 0 | 6. 0 | 12.0 | 1.0 |
| Calcium hydroxide | 38. 5 | 38. 3 | 36. 5 | 26. 5 | 14. 5 | 37. 50 |
| Polyethylene glycol | 40.0 | 40. 0 | 40.0 | 40. 0 | 40.0 | 40.0 |
| Propylene glycol | - | - | - | - | - | - |
| Glycerol | - | - | - | - | - | - |
| Barium sulfate | 12.0 | 12. 0 | 12.0 | 12.0 | 12.0 | 12. 0 |
| Titanium oxide | 6. 0 | 6. 0 | 6. 0 | 6. 0 | 6. 0 | 6. 0 |
| Aluminum oxide ultrafine particles | 3. 5 | 3. 5 | 3. 5 | 3. 5 | 3.5 | 3. 5 |
| Purified water/lithium carbonate | 1. 0 | 1. 0 | 1. 0 | 1. 0 | 1. 0 | 1.0 |
| Periapical lesion healing promoting effect | ○ (periapical lesion shrunk) | ○ (periapical lesion shrunk) | ○ (periapical lesion shrunk) | - | ○ (periapical lesion shrunk) | × (periapical lesion did not shrink) |
| Lithium elution from apical foramen | ○ (eluted 0.02 µg) | ○ (red flame was observed) | ○ (eluted 2.2 µg) | ○ (red flame was observed) | ○ (eluted 11.3 µg) | × (red flame was not observed) |
| Consistency | ○ (33.7mm) | ○ (34. 7mm) | ○ (31. 4mm) | ○ (25. 7mm) | ○ (25. 1mm) | ○ (34. 2mm) |
| Film thickness | ○ (36. 5 *µ* m) | ○ (39. 5 *µ* m) | ○ (41. 6 *µ* m) | ○ (34. 3 *µ* m) | ○ (38. 4 *µ* m) | ○ (28. 4 *µ* m) |
| Push-out resistance from syringe container | ○ (21. 4N) | ○ (21. 2N) | ○ (29. 5N) | ○ (36. 5N) | ○ (27. 5N) | ○ (23. 2N) |
| Operability | ○ (capable of being caught) | ○ (capable of being caught) | ○ (capable of being caught) | ○ (capable of being caught) | ○ (capable of being caught) | ○ (capable of being caught) |
| Stability (separation) | ○ (without separation) | ○ (without separation) | ○ (without separation) | ○ (without separation) | ○ (without separation) | ○ (without separation) |

**Table 2**

| (unit: % by weight) | | | | | | |
|---|---|---|---|---|---|---|
| | Ratio of water to lithium salt | | | | | |
| | Example 6 | Example 7 | Example 8 | Example 9 | Example 10 | Example 11 |
| Lithium carbonate | 12. 0 | 12. 0 | 12. 0 | 12.0 | 12. 0 | 12.0 |
| Lithium chloride | - | - | - | - | - | - |
| Purified water | 3.8 | 7. 2 | 12.0 | 18. 0 | 24. 0 | 36. 0 |
| Calcium hydroxide | 25.0 | 21.6 | 16.8 | 10.8 | 4.8 | 25. 0 |
| Polyethylene glycol | 37. 7 | 37. 7 | 37. 7 | 37. 7 | 37. 7 | 5. 5 |
| Propylene glycol | - | - | - | - | - | - |
| Glycerol | - | - | - | - | - | - |
| Barium sulfate | 12.0 | 12.0 | 12.0 | 12.0 | 12. 0 | 12. 0 |
| Titanium oxide | 6. 0 | 6. 0 | 6. 0 | 6. 0 | 6. 0 | 6.0 |
| Aluminum oxide ultrafine particles | 3. 5 | 3. 5 | 3. 5 | 3. 5 | 3. 5 | 3.5 |
| Purified water/lithium carbonate | 0. 3 | 0. 6 | 1. 0 | 1. 5 | 2. 0 | 3. 0 |
| Periapical lesion healing promoting effect | - | - | - | - | - | - |
| Lithium elution from apical foramen | ○ (red flame was observed) | ○ (red flame was observed) | ○ (red flame was observed) | ○ (red flame was observed) | ○ (red flame was observed) | ○ (red flame was observed) |
| Consistency | × (22.9mm) | × (20. 1mm) | ○ (25.4mm) | ○ (31.6mm) | ○ (36.2mm) | × (23. 3mm) |
| Film thickness | × (66. 1 *µ* m) | ○ (40. 3 *µ* m) | ○ (44. 5 m) | ○ (30. 8 *µ* m) | ○ (36. 2 *µ* m) | ○ (47. 2 *µ* m) |
| Push-out resistance from syringe container | × (46. 4N) | ○ (34. 2N) | ○ (26. 7N) | ○ (17. 4N) | ○ (10. 2N) | ○ (14. 8N) |
| Operability | ○ (capable of being caught) | ○ (capable of being caught) | ○ (capable of being caught) | ○ (capable of being caught) | ○ (capable of being caught) | × (putty-like) |
| Stability (separation) | ○ (without separation) | ○ (without separation) | ○ (without separation) | ○ (without separation) | ○ (without separation) | ○ (without separation) |

**Table 3**

| (unit: % by weight) | | | | | | | |
|---|---|---|---|---|---|---|---|
| | Type of lithium salt | Polyhydric alcohol concentration | | | | Without calcium hydroxide | |
| | Example 12 | Example 13 | Example 14 | Example 15 | Example 16 | Example 17 | Example 18 |
| Lithium carbonate | - | 12. 0 | 12. 0 | 0. 05 | 0.05 | 12. 0 | |
| Lithium chloride | 0. 05 | - | - | - | - | - | 20. 0 |
| Purified water | 0.05 | 48.0 | 36. 0 | 0. 05 | 0.05 | 12. 0 | 60. 0 |
| Calcium hydroxide | 38. 4 | 33. 0 | 30. 0 | 42. 9 | 2.9 | - | - |
| Calcium carbonate | - | - | - | - | - | 14. 5 | - |
| Polyethylene glycol | 40. 0 | 5.0 | 20. 0 | 50. 0 | 90.0 | 40.0 | 20. 0 |
| Propylene glycol | - | - | - | - | - | - | - |
| Glycerol | - | - | - | - | - | - | - |
| Barium sulfate | 12.0 | - | - | - | - | 12.0 | - |
| Titanium oxide | 6.0 | - | - | - | - | 6.0 | - |
| Aluminum oxide ultrafine particles | 3. 5 | 2.0 | 2.0 | 7. 0 | 7.0 | 3. 5 | - |
| Purified water/lithium carbonate | 1.0 | 4.0 | 3.0 | 1.0 | 1. 0 | 1.0 | 3.0 |
| Periapical lesion healing promoting effect | - | - | - | - | - | - | ○ (periapical lesion shrunk) |
| Lithium elution from apical foramen | ○ (red flame was observed) | ○ (red flame was observed) | ○ (red flame was observed) | ○ (red flame was observed) | ○ (red flame was observed) | ○ (red flame was observed) | ○ (red flame was observed) |
| Consistency | ○ (34.3mm) | ○ (33.1mm) | ○ (36. 2mm) | ○ (36. 0mm) | ○ (49. 6mm) | ○ (29. 3mm) | × (17. 7mm) |
| Film thickness | ○ (36. 6 *µ* m) | × (83. 7 *µ* m) | × (62. 1 *µ* m) | ○ (30. 7 *µ* m) | ○ (7. 0 *µ* m) | ○ (42. 4 *µ* m) | × (64. 2 *µ* m) |
| Push-out resistance from syringe container | ○ (26.9N) | ○ (9. 2N) | ○ (8.5N) | ○ (17.2N) | ○ (8.7N) | ○ (21. 0N) | × (90. 2N) |
| Operability | ○ (capable of being caught) | × (putty like) | × (putty like) | ○ (capable of being caught) | ○ (capable of being caught) | ○ (capable of being caught) | × (putty like) |
| Stability (separation) | ○ (without separation) | ○ (without separation) | ○ (without separation) | ○ (without separation) | ○ (without separation) | ○ (without separation) | × (solid-liquid separated) |

**Table 4**

| (unit: % by weight) | | | | | | |
|---|---|---|---|---|---|---|
| | Type of polyhydric alcohol | | Amount of ultrafine particles | | Without water | Without polyhydric alcohol |
| | Example 19 | Example 20 | Example 21 | Example 22 | Example 23 | Example 24 |
| Lithium carbonate | 12. 0 | 12. 0 | 0. 05 | 0. 05 | 12. 0 | 12. 0 |
| Lithium chloride | - | - | - | - | - | |
| Purified water | 12. 0 | 12. 0 | 0. 05 | 0. 05 | - | 48.0 |
| Calcium hydroxide | 16. 8 | 16. 8 | 29. 9 | 49. 9 | 28. 8 | 18. 5 |
| Polyethylene glycol | - | - | 50. 0 | 50. 0 | 37. 7 | - |
| Propylene glycol | 37. 7 | - | - | - | - | - |
| Glycerol | - | 37. 7 | - | - | - | - |
| Barium sulfate | 12. 0 | 12. 0 | - | - | 12. 0 | 12.0 |
| Titanium oxide | 6. 0 | 6. 0 | - | - | 6. 0 | 6. 0 |
| Aluminum oxide ultrafine particles | 3. 5 | 3. 5 | 20. 0 | - | 3. 5 | 3. 5 |
| Purified water/lithium carbonate | 1. 0 | 1. 0 | 1.0 | 1. 0 | - | 4. 0 |
| Periapical lesion healing promoting effect | - | - | - | - | - | - |
| Lithium elution from apical foramen | ○ (red flame was observed) | ○ (red flame was observed) | ○ (red flame was observed) | ○ (red flame was observed) | ○ (red flame was observed) | ○ (red flame was observed) |
| Consistency | ○ (36. 5mm) | ○ (37. 7mm) | ○ (39. 5mm) | ○ (38. 7mm) | × (24. 7mm) | ○ (28. 7mm) |
| Film thickness | ○ (48. 7 *µ* m) | ○ (31.1 *µ* m) | ○ (28. 6 *µ* m) | ○ (44. 1 *µ* m) | × (52. 8 *µ* m) | × (128. 4 *µ* m) |
| Push-out resistance from syringe container | ○ (13. 9N) | ○ (21. 3N) | ○ (36. 5N) | ○ (20. 7N) | × (10. 5N) | ○ (16. 1N) |
| Operability | ○ (capable of being caught) | ○ (capable of being caught) | ○ (capable of being caught) | ○ (capable of being caught) | ○ (capable of being caught) | × (putty-like) |
| Stability (separation) | ○ (without separation) | ○ (without separation) | ○ (without separation) | ○ (without separation) | ○ (without separation) | ○ (without separation) |

**Table 5**

| (unit: % by weight) | | |
|---|---|---|
| Components of paste A | Formulation 1 | Formulation 2 |
| Isostearic acid | 40. 0 | 39. 0 |
| Bismuth subcarbonate | 56. 0 | - |
| Silicon dioxide ultrafine particles | 4. 0 | - |
| Rosin | - | 54. 0 |
| Ester gum | - | 5. 0 |
| Benzoic acid | - | 2. 0 |

**Table 6**

| (unit: % by weight) | | | | | |
|---|---|---|---|---|---|
| Components of paste B | Formulation 1 | Formulation 2 | Formulation 3 | Formulation 4 | Formulation 5 |
| Lithium carbonate | 1.0 | 2. 0 | 4. 0 | 8. 0 | 12. 0 |
| Magnesium oxide | 6.9 | 6. 9 | 14.4 | 13. 8 | 13. 2 |
| Polyethylene glycol | 31. 7 | 31. 4 | - | - | - |
| Purified water | 4.0 | 3. 9 | - | - | - |
| Powder glass formulation 1 | 53. 5 | - | - | - | - |
| Powder glass formulation 2 | - | 52.9 | - | - | - |
| Silicon dioxide ultrafine particles | 3.0 | 2. 9 | - | - | - |
| Olive oil | - | - | 20. 2 | 19. 3 | 18. 5 |
| Zinc oxide | - | - | 46. 1 | 44. 2 | 42.2 |
| Bismuth subcarbonate | - | - | 15.4 | 14. 7 | 14. 1 |

**Table 7**

| (unit: % by weight) | | |
|---|---|---|
| Powder glass | Formulation 1 | Formulation 2 |
| CaO | 52. 0 | 54.0 |
| SiO₂ | 38.0 | 36. 0 |
| PzOs | 10.0 | 10.0 |
| CaO/SiO₂ ratio | 1.37 | 1.50 |

**Table 8**

| (unit: % by weight) | | | | | |
|---|---|---|---|---|---|
| | Example 25 | Example 26 | Example 27 | Example 28 | Example 29 |
| Combination of paste A and paste B | Formulation A1/formulation B1 | Formulation A1/ formulation B2 | Formulation A2/ formulation B3 | Formulation A2/ formulation B4 | Formulation A2/ formulation B5 |
| Lithium elution from apical foramen | ○ (eluted 0.3 µg) | ○ (red flame was observed) | ○ (eluted 0.06 µ g) | ○ (red flame was observed) | ○ (red flame was observed) |
| Consistency | ○ (25. 3mm) | ○ (24. 6mm) | ○ (31. 2mm) | ○ (31. 3mm) | ○ (30. 4mm) |
| Film thickness | ○ (26. 9 *µ* m) | ○ (27. 8 *µ* m) | ○ (18. 3 *µ* m) | ○ (15. 1 *µ* m) | ○ (17. 0 *µ* m) |
| Hardening time | ○ (180 min) | ○ (180 min) | ○ (150 min) | ○ (150 min) | ○ (150 min) |
| Operability | ○ (good string) | ○ (good string) | ○ (good string) | ○ (good string) | ○ (good string) |
| Stability of paste B (separation) | ○ (without separation) | ○ (without separation) | ○ (without separation) | ○ (without separation) | ○ (without separation) |

### (2) Evaluation method

As the compositions shown in Tables 1 to 4, 1 mL of the paste was loaded in a plastic syringe container. The paste A having a formula shown in Table 5 and the paste B having a formula shown in Table 6 were used in an equal amount in a combination shown in Table 8 and were mixed. Each product was subjected to the following tests.

### <Healing promoting effect on periapical lesion>

In accordance with the following procedure, each periapical lesion healing promoting effect of the root canal filling material compositions in Examples 1 to 3, 5, and 18 and Comparative Example 1 was examined by using four rats in each group.
1. The occlusal surface of a mandibular first molar of a 10-week-old male Wister rat under general anesthesia was cut with a round bur (#1/2) to expose the dental pulp.
2. With a K file (#10), the root apex was penetrated. By exposing the penetrated root apex to the oral cavity, a periapical lesion was formed after 4 weeks.
3. Four weeks after the dental pulp exposure, the rat was subjected to general anesthesia, and a working length was set by using an electric apex locator with rubber dam isolation of the mandibular first molar having the exposed dental pulp.
4. The root canal was enlarged with K files (#15 and #20) while being washed with a sodium hypochlorite solution.
5. The root canal irrigation was performed with an aqueous sodium hypochlorite solution in combination with ultrasonic vibration, and the root canal was dried with a paper point.
6. In the root canal, a root canal filling material as the test sample was filled and was allowed to reach the root apex by application of ultrasonic vibration.
7. A composite resin was filled to seal the dental pulp cavity.
8. To prevent the test tooth from fracturing, the maxillary first molar was extracted.
9. A week, two weeks, three weeks, and four weeks after applying the test sample into the root canal, X-ray micro-CT images were recorded, and the periapical lesion volumes were measured.

### <Elucidation of periapical lesion control mechanism>

The same procedure as in <Healing promoting effect on periapical lesion> was performed except that mice were used in place of the rats, and tissue sections of periapical lesions were prepared. As the root canal filling material composition applied into a root canal, the compositions in Example 18 and Comparative Example 1 were used.

In-situ hybridization was performed to identify the promotion of differentiation of dental pulp stem cells into odontoblasts as a factor of periapical lesion healing promotion.

Specifically, after deparaffinization of a paraffin section, the section was washed with 0. 01M PBS, then fixed with 4% PFA for 10 minutes, and was re-washed with PBS . The section was reacted with 1 µg/mL protease K (Takara Bio) for 5 minutes and was subjected to postfixation with 4% PFA. The section was subjected to acetylation treatment with 0.1M triethanolamine containing 0.25% acetic anhydride and was washed with 0.01M PBS. The section was subjected to prehybridization with a cRNA probe (Axin2: Addgene #21277, NM_015732, nt1774-2787, Col1a1: NM_007742, nt29553415, Runx2: AF010284, nt922_1746) at 55°C for 1 hour and was allowed to stand in a 5-fold concentration of sodium citrate solution (5-SSC) for 20 minutes. The section was reacted with 0.2-SSC at 70°C for 20 minutes and was allowed to stand in 0.2-SSC for 5 minutes and in a maleate buffer (MBA) for 5 minutes. The section was then subjected to blocking for 2 hours with a blocking solution containing 5% goat serum (Vector Laboratories, California, USA) . The section was reacted with an alkaline phosphatase (AP)-labelled anti-digoxigenin antibody (1:5000) (Roche, Basel, Switzerland) at 4°C for 24 hours, then was washed with an MBA containing 0.1% Tween 20, and was washed with a deionized distilled water containing 0.1% Tween 20. The section was reacted with BM Purple AP (Roche) as a substrate at room temperature for 6 hours, then was washed with PBS, next mounted with 50% glycerol/PBS, and was observed under an optical microscope.

Separately, immunofluorescence staining was performed with a cell surface marker on B cells to identify the activation of immune response, which also promotes the periapical lesion healing.

Specifically, after deparaffinization of a paraffin section, the section was washed with tris buffered saline (TBS), then was reacted in a citrate buffer at 100°C for 10 minutes, and was allowed to stand for 30 minutes at room temperature. The section was subjected to blocking with a blocking solution containing 10% goat serum (10% goat serum/TBS) for 1 hour and then was reacted with a primary antibody at room temperature overnight. CD45R (Abcam) as the primary antibody was subjected to the experiment at an antibody concentration of 1:50. After the reaction with the primary antibody, the section was washed with TBS, then was reacted with 1:500; Alexa488-goat anti-rat TgG (Invitrogen, California, USA) at room temperature for 2 hours, and was washed. The section was reacted with 1:1000; DAPI (4',6-diamidino-2-phenylindole) (Sigma) for 15 minutes. After the reaction, the section was washed and mounted with 50% glycerol. The stained tissue section was observed under a fluorescence microscope (Axioskop 2 plus: Carl Zeiss, Aalen, Germany) .

### <Lithium elution test from apical foramen>

A root canal model (END1001-30 #20, NISSIN) was enlarged to form a canal having a foramen size of 0.6 mm and a root canal volume of 0.03 mL. About 0.05 g of each composition shown in Tables 1 to 4 was filled in the root canal. As the combination compositions shown in Table 8, the paste A and the paste B were used in an equal volume and were mixed, then the mixture was loaded in a plastic syringe, and about 0.05 g of the mixture was filled in the root canal. The upper part of the root canal of each sample was sealed with an adhesive cellophane tape. Each sample was placed in a plastic container, then 10 mL of purified water was added so as to immerse the root apex, and the whole was allowed to stand in an incubator at 37°C. After each sample was stored for 7 days, the immersion liquid was collected and placed in an evaporating dish and was allowed to stand at 110°C for 1 hour 30 minutes to be concentrated. The tip of a platinum wire was immersed in the concentrated liquid, then was gently pulled up, and was placed in a colorless flame. When the flame color was observed through a cobalt glass, a composition giving a red flame was evaluated as acceptance (o), and a composition not giving a red flame was evaluated as failure (×).

For the compositions in Examples 1, 3, 5, 25, and 27, the lithium elution amount in the immersion liquid was measured by ICP-AES, and the absolute daily amount (g) of lithium eluted from the root apex was calculated. A composition having a daily lithium elution amount of 0.014 µg or more was evaluated as acceptance (∘), and a composition not having a daily lithium elution amount of 0.014 µg or more was evaluated as failure (×).

### <Flow>

For the compositions shown in Tables 1 to 4, 75 µL of the paste was interposed between glass plates (a thickness of 5 mm, 70 × 70 mm) , then a load of 2.5 kg was applied for 7 minutes, and the maximum distance and the minimum distance between parallel tangents contacting to the spread paste were measured. A composition having a flow (spreading average) of 25 to 50 mm was evaluated as acceptance (∘), and a composition not having a flow of 25 to 50 mm was evaluated as failure (×).

For the combination compositions shown in Table 8, the test was performed with reference to the test method provided in JIS-T6522-Dental root canal sealing materials. In other words, the paste A and the paste B were used in an equal volume and were mixed, and after 3 minutes, the mixture in an amount equivalent to 0.05 mL was interposed between glass plates. A weight of 120 g (including the glass plate weight) was applied for 7 minutes in an environment at 23°C and a relative humidity of 50%, and the diameter of the spread sample was measured. A composition having a paste flow of 20 to 35 mm was evaluated as acceptance (o), and a composition not having a paste flow of 20 to 35 mm was evaluated as failure (×).

### <Film thickness (JIS-T6522)>

As an index of the hardness of a paste, the test method provided in JIS-T6522-Dental root canal sealing materials was used as reference. For the compositions shown in Tables 1 to 4, about 0.07 g of the paste was interposed between glass plates (a thickness of 5 mm or more, a contact surface area of 200 ± 25 mm²), and a load of 150 ± 3 N was applied for 7 minutes by using a compact table-top precision universal tester. For the combination compositions shown in Table 8, the paste A and the paste B were used in an equal volume and were mixed, then about 0.07 g of the mixture was interposed between glass plates, and 3 minutes after the start of mixing, a load of 150 ± 3 N was applied for 7 minutes by using a compact table-top precision universal tester. The total thickness of the two glass plates and the paste film was measured, and the difference in thickness (film thickness) was calculated from the thickness without any paste film. A composition having a film thickness of not more than 50 µm was evaluated as acceptance (∘), and a composition having a film thickness of more than 50 µm was evaluated as failure (×).

### <Setting time (JIS-T6522)>

The compositions shown in Tables 1 to 4 do not harden, and thus the test was not performed. For the combination compositions shown in Table 8, the test was performed with reference to the test method provided in JIS-T6522-Dental root canal sealing materials. In other words, the paste A and the paste B were used in an equal volume and were mixed, and the mixture was filled in a mold having a diameter of *Φ* 10 mm and a height of 2 mm. From 2 minutes after mixing, the mold was placed in an environment at 37 °C and a relative humidity of 95% or more, and whether a 100g Vicat needle having a diameter of *φ* 2 mm penetrated or not was examined at predetermined time intervals. A composition having a setting time of 6 hours or less was evaluated as acceptance (o), and a composition not having a setting time of 6 hours or less was evaluated as failure (×).

### <Operability>

For the compositions shown in Tables 1 to 4, about 0.1 g of the paste was placed on a mixing paper pad and was allowed to stand in a room (a room temperature of 23 ± 1°C, a relative humidity of 50 ± 5%) for 10 minutes, and whether the paste was able to be caught on a lentulo was visually observed. A composition capable of being caught was evaluated as acceptance (∘), and a composition incapable of being caught was evaluated as failure (×).

For the combination compositions shown in Table 8, the paste A and the paste B were used in an equal volume and were mixed, then the mixture was pulled up with a spatula, and a string of the mixture was visually observed. A composition giving a string was evaluated as acceptance (o), and a composition giving no string was evaluated as failure (×).

### <Push-out test from syringe container>

For the compositions shown in Tables 1 to 4, 1 mL of the paste was packed in a plastic syringe container, and 3 days after the loading, a 21-gauge blunt needle was attached to the tip of the plastic syringe container. The syringe was displaced to 10 mm at a crosshead speed of 20 mm/min by using a compact table-top precision universal tester, and whether the paste was easily discharged from the syringe container was observed. A composition having a resistance value of not more than 40 N was evaluated as acceptance (o), a composition having a resistance value of more than 40 N was evaluated as failure (×).

### <Paste stability test>

About 1 g of each of the compositions shown in Tables 1 to 4 and the compositions (paste B) shown in Table 6 was placed in a 2-mL glass screw tube and was allowed to stand for a week at room temperature, and whether the composition separated or not was observed. A composition without separation was evaluated as acceptance (o), and a composition with separation was evaluated as failure (×).

### (3) Results

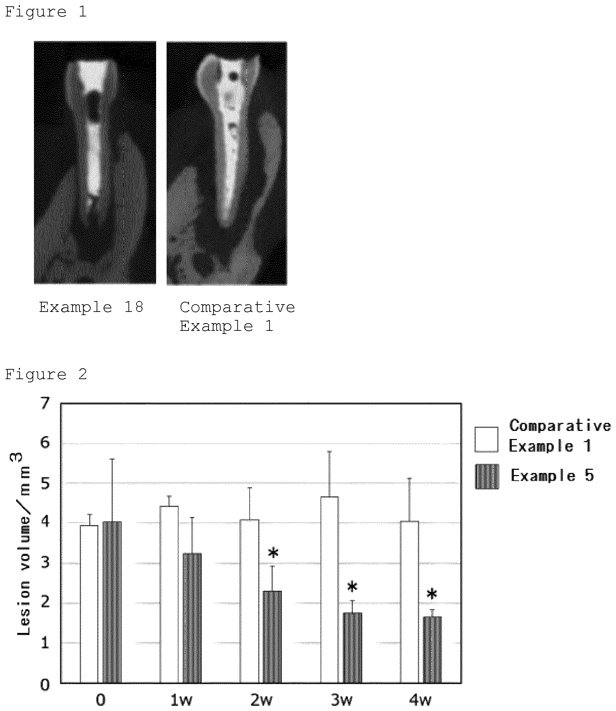

The results are shown in Tables 1 to 4, Table 8, and Figs. 1 to 3.

As shown in Table 1, the compositions in Examples 1 to 5 differed in lithium salt concentration, and each composition in the apical foramen of a root canal model eluted lithium salt. Each composition had an appropriate flow for application from a syringe container directly into a root canal and had a satisfactory film thickness, push-out properties from a syringe container, and operability. Even when each composition was stored at room temperature for a week, no separation was observed.

In Examples 6 to 11 in Table 2, the ratio of water/lithium salt was varied.

Each of the compositions in Examples 8 to 10 easily eluted lithium salt from an apical foramen, had an appropriate flow for application from a syringe container directly into a root canal, and had a satisfactory film thickness, push-out properties from a syringe container, and operability. Even when each composition was stored at room temperature for a week, no separation was observed. The composition in Example 6 contained a small amount of water relative to a lithium salt, thus was hard, and was slightly difficult to push out from a syringe container, but was usable in practice. The composition in Example 7 had poor flow, but was able to form a thin film, was easily pushed out from a syringe, did not separate, and was usable in practice. The composition in Example 11 had poor flow and turned into a putty-like substance when allowed to stand for 10 minutes, but was usable in practice when quickly handled.

The composition in Example 12 in Table 3 was the same as in Example 2 except that lithium carbonate in the composition in Example 2 was changed to lithium chloride. Regardless of the type of salt, the composition gave good performances in all the tests of the elution properties of a lithium salt from an apical foramen, the flow, the film thickness, the push-out resistance from a syringe container, the operability, and the separation.

In Examples 13 to 16 in Table 3, the concentration of a polyhydric alcohol was varied.

The compositions in Examples 13 to 16 gave substantially satisfactory results in the elution properties of a lithium salt from an apical foramen, the flow, the push-out resistance from a syringe container, and the operability. Even when each composition was stored at room temperature for a week, no separation was observed. The compositions in Examples 13 and 14 having a low polyhydric alcohol concentration gave a slightly large film thickness and turned into a putty-like substance when allowed to stand for 10 minutes, but were usable in practice when quickly handled.

The composition in Example 17 in Table 3 was the same as in Example 5 except that calcium hydroxide in the composition in Example 5 was changed to calcium carbonate. Even when containing no calcium hydroxide, the composition had a healing promoting effect on a periapical lesion and eluted lithium from the apical foramen of a root canal model. The composition had an appropriate flow for application from a syringe container directly into a root canal and had a satisfactory film thickness, push-out properties from a syringe container, and operability. Even when the composition was stored at room temperature for a week, no separation was observed.

The compositions in Examples 19 and 20 in Table 4 were the same as in Example 8 except that polyethylene glycol in the composition in Example 8 was changed to propylene glycol and glycerol, respectively. Regardless of the type of polyhydric alcohol, the compositions gave good performances in all the tests of the elution properties of a lithium salt from an apical foramen, the flow, the film thickness, the push-out resistance from a syringe container, the operability, and the separation.

The composition in Example 21 in Table 4 contained a large amount of aluminum oxide ultrafine particles, whereas the composition in Example 22 contained no aluminum oxide ultrafine particles. Regardless of the content of aluminum oxide ultrafine particles, the compositions gave good performances in all the tests of the elution properties of a lithium salt from an apical foramen, the flow, the film thickness, the push-out resistance from a syringe container, the operability, and the separation.

The composition in Example 23 in Table 4 contained no water, thus was hard, and was slightly difficult to push out from a syringe container.

The composition in Example 24 in Table 4 gave a thick film, and turned into a putty-like substance when allowed to stand for 10 minutes, but was usable in practice when quickly handled.

As shown in Table 8, the compositions in Examples 25 to 29, in which the compositions A1 and A2 as the paste A and the compositions B1 to B5 as the paste B were used in combination, had an appropriate flow and film thickness for filling in a root canal, had good setting property and operability, and eluted lithium from an apical foramen when filled in a root canal model. Separation after storage was not observed.

In Examples 1, 2, 3, 5, and 18 in which the elution of lithium from an apical foramen was observed, a decrease in volume of the lesion was observed in the evaluation of periapical lesion healing promoting effect.

Figs. 1 show X-ray micro-CT images of the left mandibular first molars 4 weeks after application of the composition in Example 18 containing a lithium salt but containing no calcium hydroxide and the composition in Comparative Example 1 containing no lithium salt. In Example 18, the radiopaque image showing the periapical lesion near the root apex shrunk, and this indicated the recovery of the bone, whereas in Comparative Example 1, the radiopaque image near the root apex remained large. In other words, in Example 18 containing a lithium salt, the lesion volume reduction effect was observed.

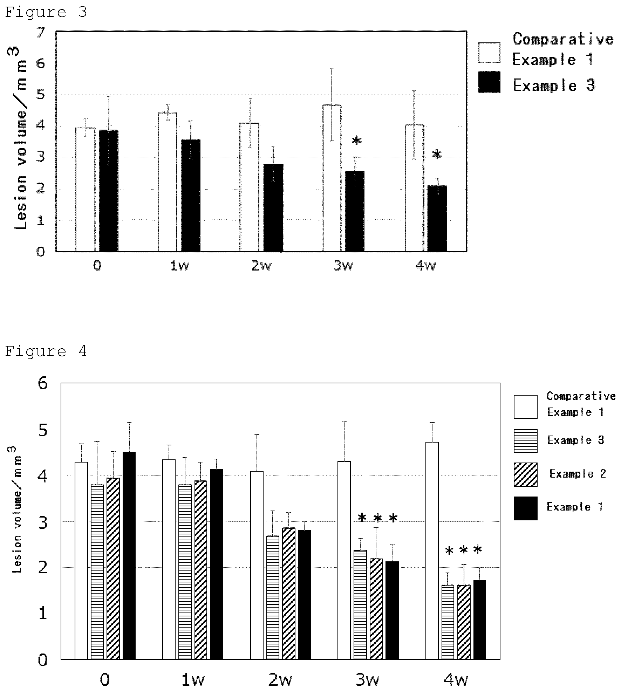

Figs. 2 to 4 show the time courses of lesion volume after application of a test composition to a root canal. As shown in Fig. 2, in Example 5 in which the lithium carbonate concentration was 12.0% by weight, the lesion volume significantly decreased after two or more weeks . In Fig. 3, Example 3 in which the lithium carbonate concentration was 1.0% by weight was compared with Comparative Example 1. As with the result in Fig. 2, in Example 3, the lesion volume significantly decreased after three or more weeks as compared with Comparative Example 1. As shown in Fig. 4, in Examples 1 to 3 in which the lithium carbonate concentration was 0.01 to 1.0% by weight, the lesion volume significantly decreased after three or more weeks as compared with Comparative Example 1 in which no lithium salt was contained. Even at an extremely small lithium salt concentration of 0.01%, the lesion volume reduction effect was sufficiently achieved. The lesion shrinking effect was not significantly affected by the lithium salt concentration.

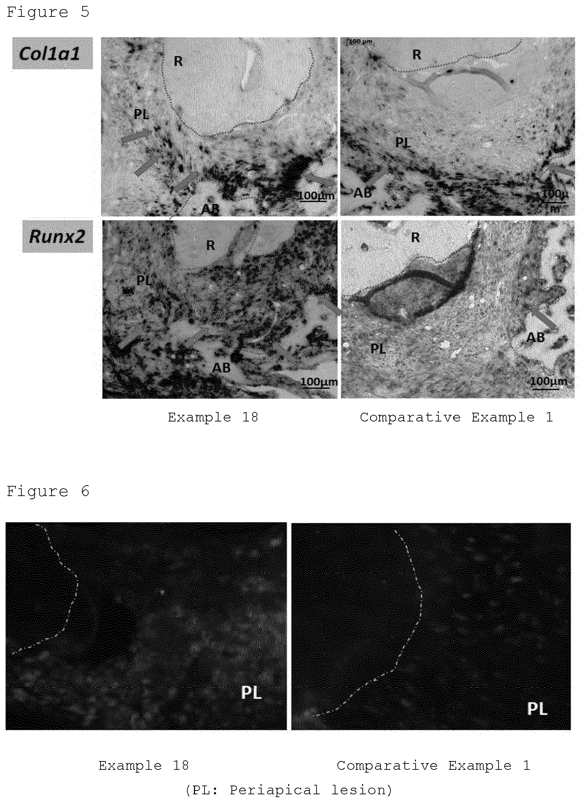

Figs. 5 show histopathological section images prepared as follows: each root canal filling material composition in Example 18 and Comparative Example 1 was filled inside an apical foramen with lesion of a mouse; and after 4 weeks, a tissue section of the periapical lesion was subjected to in-situ hybridization. The arrows indicate Colla1 or Runx2 positive cells. In Example 18, an increase in the number of Colla1 or Runx2 positive cells indicated by arrows was observed. Runx2 is a transcription factor specific to osteoblasts and regulates the differentiation of pluripotent mesenchymal cells to osteoblasts. Osteoblasts produce type I collagen that is a main component in the bone matrix. Colla1 (type I collagen α I) is one of the type I collagen. The result therefore revealed that the root canal filling material composition in Example 18 induced the differentiation from mesenchymal stem cells (dental pulp stem cells) around the root apex to osteoblasts to promote osteogenesis, which is ascribable to containing a lithium salt.

Figs. 6 show histopathological section images prepared as follows: each root canal filling material composition of Example 18 and Comparative Example 1 was filled inside an apical foramen with lesion of a mouse; and after 4 weeks, a tissue section of the periapical lesion was subjected to immunofluorescence staining with a cell surface marker on B cells. In Example 18, the number of CD45R-positive (red) cells increased as compared with Comparative Example 1. CD45R is the hematopoietic marker most abundantly expressed on all the white blood cells, and various isoforms are expressed in cell specific patterns. In mouses, CD45R/B220 isoform is expressed on B cells on various levels in all the stages mainly from pro-B cells to activated B cells. The result therefore revealed that the root canal filling material composition in Example 18 promoted the production of white blood cells to stimulate immune response, which is ascribable to containing a lithium salt.

The composition in Comparative Example 1 in Table 1 was the same as in Example 3 except that no lithium carbonate (1% by weight) was contained but the amount of calcium hydroxide increased accordingly (1% by weight).

The composition in Comparative Example 1 gave good performances in all the tests of the flow, the film thickness, the push-out resistance from a syringe container, the operability, and the separation, but provided no periapical lesion healing promoting effect, and did not elute lithium from the apical foramen of a root canal model.

### Industrial Applicability

The root canal filling material composition of the present invention comprises a lithium salt, thus can effectively promote the healing of intractable pulpitis or apical periodontitis, and is quite different from the conventional root canal filling material compositions.

## Claims

1. A root canal filling material composition comprising a lithium salt.

2. The root canal filling material composition according to claim 1, wherein a concentration of the lithium salt is 0.0001 to 20% by weight relative to a total amount of the composition.

3. The root canal filling material composition according to claim 1 or claim 2, further comprising a calcium compound.

4. The root canal filling material composition according to claim 3, wherein a concentration of the calcium compound is 2 to 70% by weight relative to a total amount of the composition.

5. The root canal filling material composition according to any one of claims 1 to 4, further comprising water.

6. The root canal filling material composition according to claim 5, wherein the water is contained 1 to 2 times the lithium salt in terms of weight ratio.

7. The root canal filling material composition according to any one of claims 1 to 6, further comprising a polyhydric alcohol.

8. The root canal filling material composition according to claim 7, wherein a concentration of the polyhydric alcohol is 1 to 90% by weight relative to a total amount of the composition.

9. The root canal filling material composition according to any one of claims 1 to 8, further comprising aluminum oxide ultrafine particles having a particle size of 1 nm to 1 µm and/or titanium oxide ultrafine particles having a particle size of 1 nm to 1 µm.

10. The root canal filling material composition according to claim 9, wherein a total content of the aluminum oxide ultrafine particles and/or the titanium oxide ultrafine particles is 0.1 to 20% by weight relative to a total amount of the composition.

11. The root canal filling material composition according to claim 1 or 2, wherein the root canal filling material composition is a combination of a paste A containing a higher fatty acid and rosin and a paste B containing magnesium oxide and a vegetable oil, and one or both of the paste A and the paste B comprise the lithium salt.

12. The root canal filling material composition according to claim 1 or 2, wherein the root canal filling material composition is a combination of a paste A and a paste B and hardens by mixing the paste A and the paste B, one or both of the paste A and the paste B comprise the lithium salt, one or both of the paste A and the paste B comprise a glass powder, the glass powder contains calcium oxide and silicon dioxide in a total amount of 50 to 100% by weight relative to a total amount of the glass powder, and in the glass powder, a weight ratio of the calcium oxide to the silicon dioxide (calcium oxide:silicon dioxide) is 6:4 to 3:7.

13. The root canal filling material composition according to claim 12, wherein a content of the glass powder is 20% by weight or more relative to a total amount of the paste A and the paste B and is 70% by weight or less relative to a total amount of each of the paste A and the paste B.

14. The root canal filling material composition according to claim 12 or 13, wherein the paste A further comprises at least one component selected from the group consisting of eugenol, guaiacol, a higher fatty acid, polyacrylic acid or a salt thereof, and phosphoric acid, and the paste B further comprises at least one component selected from the group consisting of calcium oxide, magnesium oxide, strontium oxide, zinc oxide, calcium phosphate, and aluminosilicate glass.

15. The root canal filling material composition according to any one of claims 1 to 14 for use in immunostimulation in a lesion in a root canal and/or periapical tissue.

16. The root canal filling material composition according to any one of claims 1 to 14 for use in differentiation induction from dental pulp stem cells to osteoblasts in a lesion in a root canal and/or periapical tissue.
